# FASCICULE DE BREVET EUROPEEN

(11) **EP 0 810 891 B1**
(45) Date de publication et mention de la délivrance du brevet: **10.08.2005**
(21) Numéro de dépôt: 96942387.0
(22) Date de dépôt: 11.12.1996
(51) Int. Cl.: A61M 25/00

(54) **CATHETER A SOUPAPE A FENTE(S) AXIALE(S) BI-DIRECTIONNELLE(S)**
KATHETER MIT VENTIL MIT BIDIREKTIONALEN, AXIALEN SPALTFÖRMIGEN ÖFFNUNGEN
CATHETER HAVING A VALVE WITH BIDIRECTIONAL AXIAL SLITS

(30) Priorité: 21.12.1995 FR 9515281
(43) Date de publication de la demande: 10.12.1997
(73) Titulaire: B.Braun Celsa, 86360 Chasseneuil-du-Poitou (FR)
(72) Inventeur: NADAL, Guy, F-86000 Poitiers (FR)
(74) Mandataire: Lerner, François
(86) Numéro de dépôt international: PCT/FR1996/001983
(87) Numéro de publication internationale: WO 1997/023255

(56) Documents cités:
- EP-A- 0 191 154
- EP-A- 0 328 332
- WO-A-90/09204
- FR-A- 2 707 505
- US-A- 4 029 104

## Description

L'invention se rapporte à un cathéter comportant une soupape de contrôle de fluide (particulièrement de liquide) au niveau de son extrémité distale par laquelle il est inséré dans un conduit d'un corps humain ou animal, afin de contrôler la distribution de fluide depuis le cathéter dans ledit conduit ou le prélèvement de fluide dans ce conduit.

En particulier, le cathéter de l'invention est un cathéter vasculaire propre à être inséré dans un vaisseau, de telle sorte que son extrémité distale y soit située, soit pour y prélever éventuellement du sang, soit pour y injecter un produit de traitement.

Plusieurs thérapies intraveineuses, y compris l'administration de médicaments chimiothérapeutiques et l'hyper-alimentation, exigent en effet l'utilisation d'un tel cathéter vasculaire adapté pour demeurer dans l'organisme du patient pendant une période d'implantation pouvant atteindre parfois plusieurs semaines.

Habituellement, l'implantation d'un tel cathéter s'effectue par voie fémorale ou jugulaire (à travers alors la veine sous-clavière, puis vers la veine cave supérieure).

Parmi les cathéters vasculaires existants, on connaît en particulier celui de EP-A-328 332 (ou US-A-5 030 210).

Il s'agit, comme dans l'invention, d'un cathéter "double voie" pour le contrôle de la circulation de fluide à travers lui, depuis, ou vers, un conduit d'un corps humain ou animal dans lequel le cathéter est inséré.

Ce cathéter présent un axe principal et comprend :
- un corps tubulaire allongé suivant ledit axe principal et qui présente un passage interne, un diamètre intérieur, un diamètre extérieur, et une extrémité distale ayant une ouverture communiquant avec le passage,
- un capuchon positionné par-dessus l'extrémité distale ouverte du corps et fixé à celui-ci, le capuchon présentant une paroi distale essentiellement plane située en face de l'extrémité distale ouverte du corps,
- au moins une fente formant soupape, ménagée à travers ladite paroi distale plane du capuchon, pour communiquer avec le passage du corps du cathéter, la soupape réagissant à des différences de pression de part et d'autre d'elle, pour la circulation du fluide depuis ou vers ledit conduit.

Dans le brevet susmentionné, il est indiqué que le cathéter qui y est présenté a été optimisé pour la circulation de liquide, dans le cadre d'une implantation intravasculaire.

En particulier, le problème de la circulation de liquide dans les deux sens (depuis ou vers le cathéter) y est indiqué comme ayant été prise en considération, le cathéter proposé étant censé assurer un tel fonctionnement bi-directionnel, ceci en particulier sans entraîner un pliage ou un affaissement sur lui-même du cathéter à l'aspiration.

La solution proposée prétend également pourvoir l'extrémité distale du cathéter d'une structure ayant suffisamment d'épaisseur pour ne pas se plier ou se boucher par suite des mouvements du patient, de mouvements musculaires ou d'un vide d'aspiration, la soupape à fente agencée dans une paroi relativement fine recouvrant l'extrémité distale ouverte du cathéter devant permettre en outre d'assurer une réaction rapide de la soupape aux différences de pression.

Le cathéter de EP-A-328 332, ou US-A-5 030 210, présente, a priori pour satisfaire ces exigences, un corps de cathéter de préférence en polyuréthane d'une épaisseur comprise entre environ 0,25 et 0,75 mm, le capuchon ayant quant à lui une épaisseur de paroi de préférence comprise entre 0,05 et 0,25 mm. Le caoutchouc silicone, le chlorure de polyvinyle, le polyéthylène ou le polytérafluoréthylène peuvent également être utilisés. Une seule fente formant soupape est prévue, avec une longueur comprise entre 30 et 70 % environ du diamètre interne du cathéter. Et cette fente agencée dans la paroi d'extrémité distale du capuchon est plaquée contre l'extrémité distale ouverte du corps du cathéter, de telle sorte qu'il n'existe aucun espace entre les extrémités distales de ce corps et du capuchon.

Or, il est prétendu dans le cadre de la présente invention qu'une réelle amélioration de fonctionnement, en particulier à l'aspiration, peut encore être obtenue et que la satisfaction du problème de l'affaissement sur elle-même de la paroi environnante de la soupape à fente peut également être encore améliorée, en particulier dans le cadre des cathéters vasculaires prévus pour assurer dans un sens l'injection d'un liquide de traitement vers le vaisseau et, en sens inverse, permettre le prélèvement en particulier de sang ou d'un liquide corporel.

Pour un possible fonctionnement bi-directionnel, il est en effet essentiel d'être assuré que la soupape s'ouvrira bien (dans un sens ou dans l'autre) pour une différence de pression prédéterminée et qu'elle demeurera fermée par contact entre elles de ses lèvres, si cette différence de pression n'est pas atteinte, ceci sans nuire à l'intégrité du cathéter, à sa nécessaire souplesse alliée à une tenue mécanique et à la résistance qu'il doit assurer au regard des conséquences provoqués par des mouvements inopinés du cathéter à l'intérieur de l'organisme.

Le document FR-A-2 707 505 décrit un cathéter muni d'une soupape bidirectionnelle directement fixée sur l'extrémité du cathéter et qui n'est pas renforcé pour soutenir la soupape dans le sens de circulation vers le cathéter.

La solution de l'invention est de prévoir que le cathéter présenté ci-dessus en relation avec EP-A-328 332 présente un capuchon dont la(les) fente(s) a(ont) une longueur au moins égale au diamètre intérieur du corps du cathéter. Une autre caractéristique utile également pour résoudre en particulier le problème de l'affaissement sur elle-même des parois environnantes de la fente formant soupape, notamment à l'aspiration, est que la paroi distale plane du capuchon soit située à distance de l'extrémité distale du corps du cathéter.

En particulier avec une telle caractéristique, et si la paroi distale du capuchon se présente comme un disque circulaire plein fendu, il va être possible d'allonger la(les) fente(s) de telle sorte que la longueur de fente(s) soit supérieure au diamètre extérieur de cette paroi. Il a en effet été constaté qu'un très bon fonctionnement est obtenu avec une longueur de fente(s) (en particulier deux fentes en croix s'étendant suivant deux directions perpendiculaires) comprise (par fente) entre 1,5 et 2,5 fois le diamètre intérieur du cathéter, c'est-à-dire en fait bien supérieure au diamètre extérieur du capuchon, dès lors que ce diamètre extérieur n'est que de quelques dixièmes de mm (voire éventuellement quelques mm) supérieur au diamètre intérieur du corps du cathéter.

Un problème que s'est également attaché à résoudre l'invention concerne le débit de fluide devant pouvoir passer à travers la soupape. En effet, il a été constaté que les cathéters "double voie" existants n'autorisent que des débits trop faibles pour répondre aux besoins exprimés par les utilisateurs. L'invention a donc posé ce problème, en s'imposant en outre que le débit à travers la soupape soit comparable à celui qui existerait pour un même cathéter, mais sans capuchon, et pour une même pression. A noter également que pendant la conception du présent cathéter, il s'est avéré que le problème à résoudre était lié à la manière de réaliser la soupape, ainsi qu'à certaines caractéristiques du capuchon.

La solution de l'invention est définie par les revendications 1 à 8.

A noter que l'expression "comparable" doit s'interpréter comme indiquant que le diamètre intérieur du capuchon peut être sensiblement égal (à 2 ou 3 dixièmes de mm près) à celui du corps du cathéter.

En tant que caractéristique de construction du cathéter de l'invention, on peut également noter que celles-ci sont tout à fait importantes en fonction du type d'implantation retenue, ces caractéristiques étant présentées dans la description qui suit, faite en référence aux dessins annexés dans lesquels :
- la figure 1 est une vue en coupe longitudinale d'un cathéter conforme à l'invention,
- la figure 2 est une vue de face, dans le sens de la flèche II de la figure 1 (avec complément de la coupe de cette figure 1, par symétrie),
- la figure 3 est une vue du seul capuchon toujours en coupe comme sur la figure 1,
- la figure 4 illustre schématiquement, en coupe longitudinale, la déformation que peut subir le capuchon à l'aspiration,
- la figure 5 est une vue (complétée par symétrie), de face suivant la flèche V de la figure 4,
- et la figure 6 est, comme les figures 2 et 5, une vue de face, mais d'un cathéter à fente double en croix formant soupape distale.

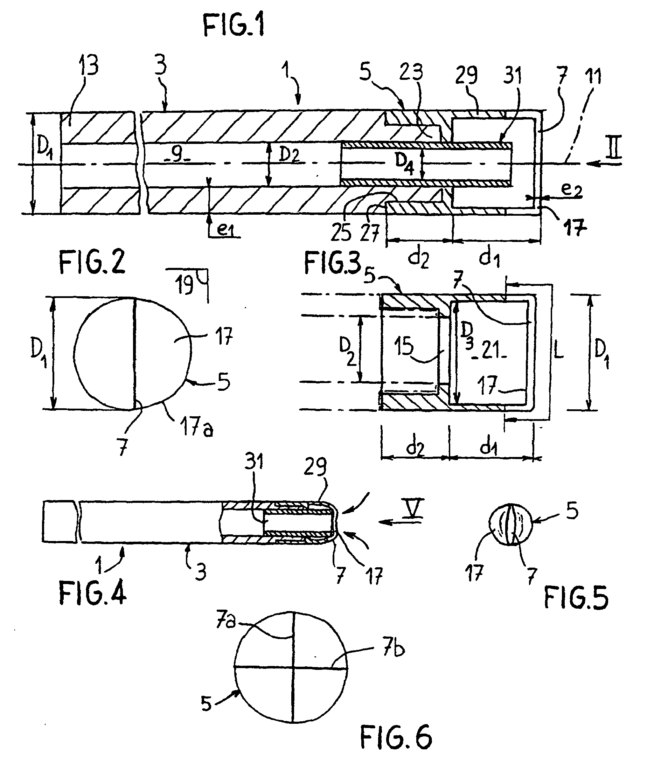

Sur la figure 1 tout d'abord, le cathéter de l'invention, repéré dans son ensemble 1, comprend un corps de cathéter 3 associé à un capuchon 5 dans la paroi duquel est ménagée une soupape bi-directionnelle à fente(s) 7.

Le corps 3 est un tube-cathéter flexible présentant une extrémité proximale ouverte 13 et une extrémité distale également ouverte, 23.

Intérieurement, le tube-cathéter 3 présente un passage 9 s'étendant suivant l'axe principal 11 du cathéter, entre les extrémités proximale et distale, ce passage se terminant par des ouvertures, telle celle repérée 15 du côté distal.

Le capuchon 5 est installé par-dessus cette ouverture 15.

Comme le corps 3, le capuchon 5 se présente ici essentiellement comme un cylindre tubulaire de section circulaire. Mais, à la différence du corps, il présente une paroi libre d'extrémité frontale, ou distale, 17 (voir figure 2). Cette paroi distale est essentiellement plane et présente un bord périphérique circulaire avantageusement arrondi 17a, atraumatique.

De préférence, le plan 19 dans lequel s'étend, au moins essentiellement, la paroi distale 17 est sensiblement perpendiculaire à l'axe principal 11 du corps et du cathéter dans son ensemble, évitant ainsi de favoriser un sens de fonctionnement de la soupape par rapport à l'autre.

C'est à travers toute l'épaisseur de cette fine paroi distale qu'est ménagée la soupape 7, et ce de façon sensiblement centrée. Sur les figures 1 à 5, il s'agit d'une fente unique. Mais plusieurs fentes, telles que deux fentes en croix comme sur la figure 6, peuvent être préférées.

Pour un fonctionnement optimal, la soupape à fente(s) 7 sera située à une distance axiale d1 de l'extrémité distale 23 du corps 3. Ainsi, le capuchon 5 présentera une sorte de chambre intérieure cylindrique 21.

Pour sa fixation, le capuchon 5 engage en outre une surface 25 du tube-cathéter s'étendant sur une distance axiale d2 à partir de l'extrémité 23, en direction de l'extrémité 13.

Ainsi, sur la version de la figure 1, le capuchon peut être considéré comme comprenant deux parties, l'une de fixation au tube 3 (et de longueur d2), l'autre, de longueur d1, destinée au fonctionnement optimal bi-directionnel de la soupape 7.

Le diamètre extérieur du cathéter étant dans de nombreux cas décisif, eu égard aux conditions d'implantation dans des vaisseaux de faibles diamètres, il est préférable que le corps 3 et le capuchon 5 présentent sensiblement un même diamètre extérieur D1.

Pour cela, l'épaisseur e₁ du corps 3 est amoindrie à l'endroit de la surface 25, suite à un épaulement 27, la fixation du capuchon à l'endroit de cette surface 25 pouvant être assurée par collage, ou tout autre moyen de liaison intime.

Pour un bon fonctionnement de la soupape 7, l'épaisseur e₂ de la paroi latérale cylindrique 29 située au-delà de l'extrémité distale 23 sera avantageusement inférieure à celle de la paroi de la zone de fixation 25 et bien entendu à l'épaisseur e₁ du tube-cathéter. Ainsi, on favorisera l'équilibre recherchée entre une résistance structurelle suffisante de la partie distale du cathéter et la nécessaire souplesse dans l'environnement de la soupape.

En relation avec cela, il est d'ailleurs important dans l'invention que la(ou les) fente(s) 7 présente(nt) une longueur L (voir figure 3) supérieure ou égale au diamètre intérieur du tube 3 au moins du côté de son extrémité distale, ce diamètre étant en l'espèce constant sur toute la longueur du tube et correspondant à celui, D2, du passage 9.

En pratique, il a même été constaté qu'une optimisation du fonctionnement bi-directionnel pouvait être obtenue si la longueur de la soupape à fente(s) était au moins égale au diamètre extérieur D1, voire même avantageusement supérieure. C'est d'ailleurs pour cela que l'on peut voir en particulier sur les figures 1 et 3 que la fente 7 se prolonge au-delà de la paroi 17, sur une partie de la paroi latérale cylindrique 29.

Une longueur de fente(s) comprise (par fente) entre 1,5 et 2,5 fois le diamètre D2 est au demeurant conseillée, en particulier si la dureté du capuchon 5 est comprise entre environ 45 et 55 shores A, le diamètre extérieur commun D1 (ou au moins celui du capuchon) est de l'ordre de 2,2 mm à 2,8 mm et (de préférence environ 2,5 mm, le diamètre D2 est de l'ordre de 1,1 à 1,4 mm, le diamètre intérieur D3 du capuchon est d'environ 1,8 à 2 mm et la soupape est constituée par deux fentes perpendiculaires en croix (cf. figure 6) ayant chacune une longueur L d'environ 3 mm à 4,5 mm, et de préférence d'environ 3,8 mm à 4 mm. Avec une telle réalisation, l'épaisseur e₂ des parois 17 et 29 sera avantageusement de l'ordre de 0,3 mm, l'épaisseur e₁ de l'ordre de 0,5 mm, et la dureté du corps 3 de l'ordre de 60 à 70 shores A.

Tant le corps que le capuchon peuvent être en caoutchouc silicone, propre à une implantation vasculaire.

Selon une autre caractéristique très avantageuse de l'invention, on notera en outre en particulier sur la figure 1 que l'extrémité distale du corps 3 a été intérieurement "renforcée" par une structure 31 destinée à assurer un appui pour le capuchon lorsque la soupape 7 va fonctionner à l'aspiration.

La structure 31 illustrée est un tube inséré dans le passage 9, depuis l'extrémité distale 23.

Surtout si la soupape 7 est ménagée à distance de cette extrémité 23, le renfort 31 s'étendra de préférence également vers cette fente, à l'intérieur de la chambre 21 du capuchon.

S'il s'agit d'un tube, il pourra être en silicone ou en polyuréthane et d'une longueur de quelque mm.

Son diamètre intérieur D4 sera de préférence inférieur au diamètre D2. Quant à son diamètre extérieur, il sera sensiblement égal à D2. Ainsi, on créera entre le passage 9 et la soupape 7 (ou la chambre 21) un effet de "Venturi" favorable à la circulation du liquide avec le débit souhaité.

Lorsqu'un produit de traitement est à éjecter hors du cathéter, après avoir circulé dans le passage 9, la soupape 7 va fonctionner à l'éjection par écartement des lèvres de la fente et légère déformation de ces lèvres vers l'extérieur, sensiblement à la manière d'une bouche qui s'ouvre avec des lèvres qui s'avancent.

A l'aspiration, si un excès de pression agit sur l'extérieur de la fente, en vue d'une circulation par exemple de sang vers le passage 9, le cathéter prendra alors sensiblement la forme illustrée sur la figure 4, avec des lèvres de soupape légèrement écartées comme sur la figure 5.

Sur la figure 4, on notera en particulier que dans un tel fonctionnement à l'aspiration, les parois 17 et/ou 29 du capuchon se déforment vers l'intérieur, jusqu'à venir prendre appui sur le tube 31, évitant ainsi tout affaissement excessif du capuchon sur lui-même risquant d'empêcher un fonctionnement correct, voire même une fermeture de la fente.

La figure 6 montre, comme déjà indiqué, que la soupape à fente(s) pourrait être réalisée comme une fente multiple présentant par exemple deux parties fendues en croix, 7a, 7b. Éventuellement, plus de quatre secteurs pourraient même être prévus. Bien entendu, la (chaque) fente est réalisée comme une coupe locale à travers toute l'épaisseur de la paroi considérée, sans enlèvement de matière.

## Revendications

1. Cathéter pour le contrôle de la circulation de fluide à travers lui, depuis ou vers un conduit d'un corps humain ou animal, comme par exemple un vaisseau, dans lequel le cathéter est inséré, ledit cathéter (1) présentant un axe principal (11) et comprenant :
- un corps tubulaire (3) allongé suivant ledit axe principal et présentant un passage interne (9), un diamètre intérieur (D2), un diamètre extérieur (D1) et une extrémité distale (23) ayant une ouverture (15) communiquant avec ledit passage,
- un capuchon (5) positionné par-dessus ladite extrémité distale ouverte et fixé au corps (3) du cathéter, le capuchon présentant une paroi distale (17) essentiellement plane située en face de l'extrémité distale ouverte du corps , ladite paroi distale (17) étant située à distance de l'extrémité distale (23) du corps (3),
- au moins une fente linéaire formant soupape (7 ; 7a, 7b) ménagée à travers ladite paroi distale plane (17) du capuchon, pour communiquer avec le passage (9) du corps du cathéter, la soupape réagissant à des différences de pression de part et d'autre d'elle, pour la circulation dudit fluide depuis ou vers ledit conduit, la fente présentant une longueur (L) supérieure ou égale au diamètre intérieur (D2) du corps (3) du cathéter,
**caractérisé en ce que** le cathéter est intérieurement renforcé vers son extrémité distale, pour assurer un appui d'une paroi (17, 29) du capuchon, lorsque ladite soupape fonctionne pour une circulation du fluide depuis le conduit vers le passage (9) du corps.

2. Cathéter selon la revendication 1, **caractérisé en ce que** la paroi distale (17) du capuchon (5) présente un diamètre extérieur (D1) et la longueur (L) de la(de chaque) fente (7) est supérieure au diamètre extérieur de ladite paroi distale (17).

3. Cathéter selon l'une des revendications 1 et 2, **caractérisé en ce que** :
- le capuchon présente une paroi latérale (29) sensiblement cylindrique,
- le capuchon présente un diamètre intérieur comparable ou supérieur à celui (D2) du corps (3) du cathéter, et
- la, ou chaque, fente s'étend à la fois sur la paroi distale plane (17) du capuchon et sur sa paroi latérale cylindrique (29).

4. Cathéter selon l'une quelconque des revendications précédentes, **caractérisé en ce que** pour renforcer intérieurement le cathéter vers son extrémité distale, un tube (31) est inséré dans le passage intérieur (9) dudit corps.

5. Cathéter selon l'une quelconque des revendications précédentes, **caractérisé en ce que** le renfort intérieur (31) du corps du cathéter s'étend au-delà de l'extrémité distale (23) du corps (3) du cathéter, à l'intérieur de la partie (21) du capuchon (5) située au-delà de cette extrémité, vers la soupape.

6. Cathéter selon l'une quelconque des revendications précédentes, **caractérisé en ce que** la longueur (L) de la, ou de chaque, fente (7 ; 7a, 7b) est comprise entre 1,5 et 2,5 fois le diamètre intérieur (D2) du corps du cathéter.

7. Cathéter selon l'une quelconque des revendications 4 à 6, **caractérisé en ce que** le tube (31) présente un diamètre intérieur inférieur au diamètre intérieur (D2) du corps (3) et le capuchon (5) présente un diamètre intérieur supérieur au diamètre extérieur dudit tube.

8. Cathéter selon l'une quelconque des revendications précédentes, **caractérisé en ce que** :
- la soupape (7) comprend au moins deux fentes disposées en croix, ayant chacune une longueur comprise entre environ 3 mm et 4,5 mm, et
- le capuchon présente une dureté comprise entre environ 45 et 55 Shore A, un diamètre extérieur compris entre environ 2,2 mm et 2,8 mm, ainsi qu'un diamètre intérieur supérieur de plus de 0,3 mm environ à celui du corps (3) du cathéter.

## Patentansprüche

1. Katheter für die Überwachung der Fluidzirkulation durch ihn hindurch, von oder zu einem Gang eines menschlichen oder tierischen Körpers, wie z.B. einem Gefäß, in weiches der Katheter eingefügt ist, wobei der Katheter (1) eine Hauptachse (11) hat und aufweist:
- einen röhrenförmigen Körper (3), der entlang der Hauptachse verlängert ist und einen inneren Durchgang (9), einen inneren Durchmesser (D2), einen äußeren Durchmesser (D1) und ein distales Ende (23) aufweist, das eine Öffnung (15) hat, welche mit dem Durchgang in Verbindung steht,
- eine Kappe (5), die über dem offenen distalen Ende angeordnet und an dem Körper (3) des Katheters befestigt ist und eine im wesentlichen ebene distale Wand (17) aufweist, die gegenüber dem distalen offenen Ende des Körpers angeordnet ist und im Abstand von dem distalen Ende (23) des Körpers (3) angeordnet ist,
- mindestens einen ein Ventil (7; 7a, 7b) bildenden linearen Schlitz, der quer durch die ebene distale Wand (17) der Kappe ausgespart ist, um mit dem Durchgang (9) des Katheterkörpers in Verbindung zu stehen, wobei das Ventil auf Druckunterschiede auf seinen beiden Seiten für die Zirkulation des Fluids von oder zu dem Gang hin reagiert und der Schlitz eine Länge (L) aufweist, die größer oder gleich dem inneren Durchmesser (D2) des Körpers (3) des Katheters ist,
**dadurch gekennzeichnet, daß** der Katheter innen zu seinem distalen Ende hin verstärkt ist, um einen Halt einer Wand (17, 29) der Kappe sicherzustellen, wenn das Ventil für eine Fluidzirkulation von dem Gang zu dem Durchgang (9) des Körpers hin arbeitet.

2. Katheter nach Anspruch 1, **dadurch gekennzeichnet, daß** die distale Wand (17) der Kappe (5) einen äußeren Durchmesser (D1) aufweist, und die Länge (L) des (jedes) Schlitzes (7) größer ist als der äußere Durchmesser der distalen Wand (17).

3. Katheter nach einem der Ansprüche 1 und 2, **dadurch gekennzeichnet, daß**:
- die Kappe eine im wesentlichen zylindrische Seitenwand (29) aufweist,
- die Kappe einen inneren Durchmesser aufweist, der mit dem (D2) des Körpers (3) des Katheters vergleichbar oder größer ist, und
- sich der oder jeder Schlitz zugleich auf der ebenen distalen Wand (17) der Kappe und auf seiner zylindrischen Seitenwand (29) erstreckt.

4. Katheter nach einem der vorhergehenden Ansprüche, **dadurch gekennzeichnet, daß** ein Rohr (31) in den inneren Durchgang (9) des Körpers eingeführt ist, um den Katheter innen zu seinem distalen Ende hin zu verstärken.

5. Katheter nach einem der vorhergehenden Ansprüche, **dadurch gekennzeichnet, daß** sich die innere Verstärkung (31) des Katheterkörpers über das distale Ende (23) des Körpers (3) des Katheters im Inneren des Teils (21) der Kappe (5), der über diesem Ende angeordnet ist, zu dem Ventil hin erstreckt.

6. Katheter nach einem der vorhergehenden Ansprüche, **dadurch gekennzeichnet, daß** die Länge (L) des oder jedes Schlitzes (7; 7a, 7b) zwischen dem 1,5- und 2,5-fachen des inneren Durchmessers (D2) des Katheterkörpers beträgt.

7. Katheter nach einem der Ansprüche 4 bis 6, **dadurch gekennzeichnet, daß** das Rohr (31) einen inneren Durchmesser aufweist, der kleiner als der innere Durchmesser (D2) des Körpers (3) ist, und die Kappe (5) einen inneren Durchmesser aufweist, der größer als der äußere Durchmesser des Rohrs ist.

8. Katheter nach einem der vorhergehenden Ansprüche, **dadurch gekennzeichnet, daß**:
- das Ventil (7) mindestens zwei gekreuzte Schlitze aufweist, von denen jeder eine Länge zwischen etwa 3 mm und 4,5 mm hat, und
- die Kappe eine Härte von etwa zwischen 45 und 55 Shore A, einen äußeren Durchmesser von zwischen etwa 2,2 mm und 2,8 mm, sowie einen inneren Durchmesser aufweist, der um mehr als etwa 0,3 mm größer ist als der des Körpers (3) des Katheters.

## Claims

1. A catheter for controlling the circulation of fluid there through, from or to a duct of a human or animal body in which the catheter is inserted, the catheter (1) having a principal axis and comprising:
a tubular body (3) elongated along the principal axis and having an internal passage (9), an inside diameter (D2), an outside diameter (D1), and a distal end (23) having an opening (15) that communicates with the passage;
a cap (5) disposed over the open distal end and affixed to the body (3) of the catheter, the cap having an essentially flat distal wall (17) facing the open distal end of the body, the distal wall (17) being spaced from the distal end (23) of the body (3); and
at least a linear slit forming a valve (7; 7a, 7b) through the flat distal wall (17) of the cap, to communicate with the passage (9) in the body of the catheter, the valve reacting to pressure differences on either side thereof, for the circulation of the fluid from or to the duct, the slit having a length (L) that is greater than or equal to the inside diameter (D2) of the body (3) of the catheter;
**characterized in that** the catheter is internally strengthened near its distal end for providing support of a cap wall (17, 29) when said valve operates for circulating fluid from the duct to the body passage (9).

2. Catheter according to claim 1, **characterized in that** the distal wall (17) of the cap has an outside diameter (D1) and the length (L) of said (or each) slit is larger than the outside diameter of the distal wall (17).

3. Catheter according any one of claims 1 and 2, **characterized in that**:
the cap has a substantially cylindrical side wall (29);
the cap has an inside diameter that is substantially equal to or larger than that (D2) of the body (3) of the catheter; and
the slit or each slit extends both over the plane distal wall (17) of the cap and the cylindrical side wall (29) thereof.

4. Catheter according any one of preceding claims, **characterized in that**, for internally strengthening of the catheter near its distal end, a tube (31) is inserted in the body internal passage (9).

5. Catheter according any one of preceding claims, **characterized in that** internal strengthener (31) of the catheter body extends beyond the distal end (23) of the catheter body (3), into the portion (21) of the cap (5) disposed beyond this end towards the valve.

6. Catheter according any one of preceding claims, **characterized in that** the length (L) of the slit or each slit (7; 7a, 7b) is from 1.5 to 2.5 times the inside diameter (D2) of the body of the catheter.

7. Catheter according any one of claims 4 to 6, **characterized in that** the tube (31) has an inside diameter that is smaller than the inside diameter (D2) of the body (3), and the cap (5) has an inside diameter that is larger than the outside diameter of said tube.

8. Catheter according any one of preceding claims, **characterized in that**:
the valve (7) comprises at least two slits arranged in a cross formation, each slit having a length of approximately from 3 mm to 4.5 mm; and
the cap has a hardness of approximately 45 to 55 Shore A, an outside diameter of approximately from 2.2 mm to 2.8 mm, and an inside diameter that is more than approximately 0.3 mm larger than the inside diameter of the body (3) of the catheter.
